# EUROPÄISCHE PATENTSCHRIFT

(11) **EP 1 778 783 B1**
(45) Veröffentlichungstag und Bekanntmachung des Hinweises auf die Patenterteilung: **10.02.2010**
(21) Anmeldenummer: 05761130.3
(22) Anmeldetag: 17.06.2005
(51) Int. Cl.: C08L 23/22, C09J 123/22, A61K 9/70, C09J 153/02

(54) **WASSERHALTIGE POLYMERMATRIX AUS HYDROPHOBEN POLYMEREN**
POLYMER MATRIX CONTAINING WATER, CONSISTING OF HYDROPHOBIC POLYMERS
MATRICE POLYMERE CONTENANT DE L'EAU EN POLYMERES HYDROPHOBES

(30) Priorität: 09.08.2004 DE 102004038533
(43) Veröffentlichungstag der Anmeldung: 02.05.2007
(73) Patentinhaber: Beiersdorf AG, 20253 Hamburg (DE)
(72) Erfinder: NIERLE, Jens, 21147 Hamburg (DE)
(86) Internationale Anmeldenummer: PCT/EP2005/052815
(87) Internationale Veröffentlichungsnummer: WO 2006/015898

(56) Entgegenhaltungen:
- WO-A-02/36103
- WO-A-03/080035
- US-A- 3 036 988

## Beschreibung

Die vorliegende Erfindung betrifft Mischsysteme umfassend die Komponenten Polyvinylpyrrolidon und/oder deren Copolymere und Wasser.

Zusätzlich können Wirkstoffe in das System eingebunden sein, die in hydrophoben Matrices schlecht oder gar nicht inkorporiert werden können. Das Mischsystem ermöglicht, ansonsten schwer lösliche, pharmazeutische Wirkstoffe, z.B. Dexpanthenol, in eine unpolare Matrix einzubringen. Der Vorteil des Systems ist, dass damit polare Wirkstoffe in einer unpolaren Matrix wie z.B. Polyisobutylen über den Sättigungspunkt hinaus gelöst werden können. Darüber hinaus verhindert das Mischsystem die Kristallisation des Wirkstoffs, wodurch eine konstante Freisetzung des Wirkstoffs über die gesamte Haltbarkeitsdauer eines transdermalen Systems, wie z.B. Pflaster, gewährleistet wird.

Bei der Herstellung von Polymermatrices mit hydrophilen kosmetischen, aber auch pharmazeutischen Wirkstoffen ergibt sich oft das Problem, dass sich die Stoffe nicht ausreichend gut in die Matrix einarbeiten lassen. Sie lösen sich nur sehr begrenzt, was die Freisetzung und damit auch die Wirksamkeit stark herabsetzt.

Ein Problem bei der Herstellung transdermaler therapeutischer Systeme ist das Einbringen polarer Wirkstoffe in die zumeist unpolaren Polymermatrizes. Dadurch können bevorzugte Wirkstoffe mitunter nur schlecht oder nur in begrenzter Konzentration in die Polymermatrix eingearbeitet werden. Des weiteren besteht die Gefahr, dass aufgrund der unterschiedlichen Polarität und Unlöslichkeit der Wirkstoffe in der Polymermatrix die Wirkstoffe mit der Zeit aus dem Polymersystem auskristallisieren. Eine Langzeitstabilität ist damit nicht gewährleistet.
Einen Vorschlag zur Lösung dieser Probleme wird durch den Zusatz von Lösemittel gegeben. Die Einarbeitung von Lösemittel in eine polymere Matrix hat aber verschiedene Nachteile. Zu erwähnen sind hier die Schädlichkeit der meisten organischen Lösungsmittel, hohe technische Aufwände für Absaugung und Wiedergewinnung, hohe Kosten für erforderliche hochreine Lösungsmittel und besonders ein sehr hoher Aufwand zur Entfernung von Lösungsmittelresten aus der Matrix.

DE 10212864 beschreibt eine Polymermatrix, die neben Polyvinylpyrrolidon PVP zwingend DMSO enthält, um die Löslichkeit von polaren Wirkstoffen in unpolaren Polymermatrizes zu ermöglichen und das Auskristallisieren der Wirkstoffe in den unpolaren Polymermatrizes zu verhindern ohne dass zusätzliche Lösungsmittel eingesetzt werden.

US 3036988 offenbart Polymersysteme enthaltend Polyamide, Polyvinylpyrrolidon und Wasser um aus den Polymerisaten Garne zu extrudieren_{.}

Aufgabe der vorliegenden Erfindung ist es ein alternatives System zu entwickeln, das die Aufnahme von polaren Wirkstoffen in unpolaren Polymermatrizes ermöglicht und das Auskristallisieren der Wirkstoffe in den unpolaren Polymermatrizes verhindert.

Aufgabe der vorliegenden Erfindung ist es auch eine Polymermatrix zur Auflage auf die Haut bereit zu stellen, die wasserhaltig ist und damit an die gesunde oder geschädigte Haut Feuchtigkeit abgeben kann und/oder zu einer kühlenden Wirkung beiträgt.

Es war überraschend und für den Fachmann nicht vorauszusehen und darin liegt die Lösung dieser Aufgaben, dass man Wasser in Kombination mit PVP als Gel in eine hydrophobe Polymermatrix einarbeitet.

Dabei kann das Gel sowohl mit einem Überschuß an Wasser, als auch mit einem Überschuß an PVP verwendet werden. Man kann somit ohne weiteres 0 - 20% Wasser in eine PIB-Matrix einarbeiten. Die Polymermatrix, deren Polymere einen hydrophoben Charakter aufweisen, umfassen 0,1 bis 25 Gew,%, bezogen auf die Masse der Gesamtpolymermatrix, eines Mischsystems umfassend Poly(1-vinyl-2-pyrrolidone) der allgemeinen Formel 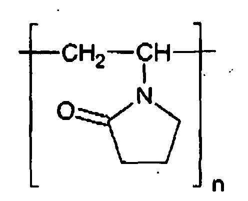 mit n = 350 bis 13500 und/oder deren Copolymere und Wasser.

Als geeignet haben sich Gewichtsverhältnisse von PVP zu Wasser von 1:5 bis 5:1, bevorzugt 1:3 bis 3:1, insbesondere 1:2 gezeigt.

Als weitere Matrices sind neben PIB Polyisobutylen hydrophobe Basispolymere wie SIS (Styrol/Isopren/Styrol)-Triblockcopolymere, SBS (Styrol/Butadien/Styrol)-Triblockcopolymere, SBR (Copolymere aus Styrol und Butadien), synthetische und/oder natürliche Polyisoprene, Polyester, Co-Polyester und/oder Mischungen daraus erfindungsgemäß. Aus der Vielzahl bekannter Polymermatrizes sind Polyisobutylene besonders bevorzugt. Polyisobutylene erfüllen als Matrixgrundlage die Anforderungen einer selbstklebenden, hautschonenden und schmerzfrei ablösbaren Polymermatrix besonders gut, so dass es folgerichtig ist, die Polyisobutylene bevorzugt als Matrixgrundlage auszuwählen.

SBR ist eine Sammeibezeichnung für Copolymere aus Styrol und Butadien, die die beiden Monomere meistens im Gewichtsverhältnis von ca. 23,5:76,5. in Ausnahmefällen auch von 40:60 enthalten und deren Makromoleküle überwiegend die Struktureinheiten I und II aufweisen:

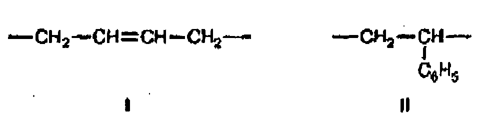

Erfindungsgemäße wasserhaltige Matrices können dazu benutzt werden um sehr trockene Hautareale mit Feuchtigkeit zu versorgen.
Damit ist die erfindungsgemäße Polymermatrix als Pflaster, Pad oder Hautauflage zur Pflege der Haut und insbesondere zu einfachen Kühlungszwecken außerordentlich gut geeignet und, zudem selbstklebend ausgerüstet, einfach anzuwenden.

Vorteilhaft ist auch, entsprechend zur Vermeidung der Nachteile aus dem Stand der Technik, dass die Polymermatrix lösemittelfrei ist.

In der Kombination der beiden Verbindungen PVP und/oder deren Copolymere und Wasser liegt der nicht vorhersehbare, vorteilhafte Schritt zur Lösung der gestellten Aufgaben, Beide Verbindungen erbringen synergistisch erst den gewünschten Effekt der möglichen Integration polarer Wirkstoffe in unpolaren Polymermatrizes. Der Einsatz jeder Verbindung alleine führt nicht zum Erfolg. Wasser lässt sich per se nicht in unpolaren Matrices einarbeiten.
PVP ist in unpolaren Polymeren, insbesondere Polyisobutylen, unlöslich und würde in Form von kugelförmigen Einschlüssen vorliegen. Erst durch die Herstellung des PVP/Wasser-Mischsystems, das vorteilhafterweise als Gel formuliert wird, ist eine vollständige Integration der beiden Komponenten in die unpolare Matrix möglich. Man stellt weder ein Austreten des Wassers aus der Matrix, noch eine Beeinträchtigung der Klebkraft fest. Darüber hinaus kann das PVP und/oder deren Copolymere Koordinationsverbindungen mit Füllstoffen eingehen, die in der Matrix enthalten sind, sofern diese polare Gruppen, wie beispielsweise Hydroxygruppen besitzen. Über die N-Funktionalität des PVP können so Wirk- und/oder Füllstoffe assoziiert werden. Zu den assoziierenden Füllstoffen können unter anderem verschiedene Cellulosetypen gewählt werden. Beispielweise lassen sich pulvrige Cellulosen, wie Elcema P050 der Fa. Degussa, als auch mikrokristalline Cellulose verwenden. Durch die Koordination des PVP an die Cellulose wird eine deutlich höhere Härte des Systems gegenüber dem PVP-freien Systemen einstellbar. Dieser Effekt führt zu einer weiteren Verbesserung der Kompatibilität des PVP/Wasser-Mischsystems mit der Polymermatrix.

PVP gilt als weitestgehend inert, so dass nicht mit chemischen Reaktionen mit den pharmazeutischen Wirkstoffen in der Polymermatrix gerechnet werden muss. Im Gegensatz zu dem aus der WO 01/68060 beschriebenen System auf Lösungsmittelbasis und Polyacrylaten ist PVP in einem Hotmelt-Prozess nicht in reiner Form einsetzbar, wenn mit unpolaren Polymeren als Matrix gearbeitet wird. Wie oben beschrieben löst sich das PVP dann nur schlecht in der Matrix. Im Prozess auf Lösungsmittelbasis wird hingegen mit Alkoholen als Lösungsmitteln gearbeitet. Diese Lösungsmittel führen zu einem Quellen des PVPs. Dadurch entfaltet der Rohstoff zwar seine filmbildenden Eigenschaften und kann homogen in die Masse eingearbeitet werden und dieser homogene Zustand bleibt auch nach dem zwingenden Entfernen des Lösungsmittels erhalten, aber die Nachteile von lösungsmittelhaltigen Systemen sind bekannt.

Das erfindungsgemäße Mischsystem aus PVP und/oder deren Copolymere und Wasser hat sehr gute Lösungseigenschaften für polare Wirkstoffe wie beispielsweise Dexpanthenol. Die gute Lösungsvermittlung des erfindungsgemäßen Mischsystems für Wirkstoffe in unpolaren Matrizes beruht einerseits darauf, dass saure Wirkstoffe an die Stickstofffunktionen des PVP koordinieren können und dass andererseits das Wasser im Gel oder Mischsystem ein sehr hohes Löslichkeitspotenzial besitzt. Die Stabilisierung des Wirkstoffes gegenüber einem Auskristallisieren aus der Matrix wird durch beide Eigenschaften synergistisch verstärkt

In diese Matrix lassen sich insbesondere Wirkstoffe wie Dexpanthenol, Ascorbinsäure und/oder Harnstoff einbinden. Es ist auch möglich weitere wasserlösliche Stoffe einzuarbeiten. Die Stoffe liegen dann vollständig gelöst vor. Durch das enthaltene Wasser wird ein auskristallisieren der Wirkstoffe verhindert.

Ein weiterer wesentlicher Vorteil der erfindungsgemäßen Polymermatrices ist, dass die Klebkraft der Matrix sich individuell auf die zu behandelnde Hautschädigung einstellen lässt, so dass auch Anwendungen auf geschädigten Hautarealen, wie zum Beispiel bei Sonnenbränden möglich werden. Dabei können zum Beispiel auch UV-Filter eingearbeitet werden, die einen Schutz der betroffenen Hautstellen gegen weitere Sonneneinstrahlung und damit weiterer Schädigung bieten. Eine Kombination aus UV-Filter und heilungsfördemdem Wirkstoff als Bestandteil der erfindungsgemäßen Matrix ist somit möglich. Eine weitere Anwendung ist der vorbeugende UV-Schutz von besonders gefährdeten Hautarealen wie zum Beispiel des Nasenrückens.
Erfindungsgemäß wird aus ästhetischen Gründen hier ein transparentes Pflaster, das nur mit einem UV-Filter dotiert ist zum Einsatz kommen.

PVP Poly(1-vinyl-2-pyrrolidone) der allgemeinen Formel 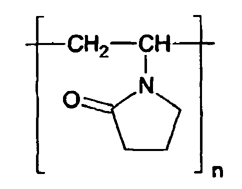 mit n = 350 bis 13500 und/oder deren Copolymere sind erfindungsgemäß Bestandteil der hydrophoben Polymermatrix. Als weiter vorteilhaft anzusehen ist, wenn Poly(1-vinyl-2-pyrrolidone) und/oder deren Copolymere ein mittleres Molekulargewicht von 2000 bis 1.500.000 g/mol, insbesondere 44.000 bis 54.000 g/mol aufweisen. Neben Kollidon 30, Luviskol 30 lassen sich auch PVP mit anderen Molekulargewichten einsetzen, wie beispielsweise Luviskol 90 oder Derivate des PVP.

Die Herstellung und Anwendung der Polymermatrix zu Pflastern, Pads oder Hautauflagen kann der Fachmann dem bekannten Stand der Technik entnehmen oder den nachfolgenden Beispielen.

Die nachfolgenden Beispiele erläutern die erfindungsgemäßen Polymermatrices sowie deren Herstellung.

### Beispiel 1:

In einem Becherglas werden 20,0g (50% w/w) Wasser vorgelegt und auf 70°C erwärmt. Unter Rühren werden 10,0g (50% w/w) Kollidon 30^{®} hinzugefügt. Die Mischung wird 45min bei 70°C gerührt. Die fertige Mischung liegt als Gel vor, wird abgekühlt und in ein Vorratsgefäß abgefüllt.

### Beispiel 2:

In einem Becherglas werden 20,0g (50% w/w) Wasser vorgelegt und auf 70°C erwärmt. Unter Rühren werden 10,0g (50% w/w) Kollidon 90^{®} hinzugefügt. Die Mischung wird 45min bei 70°C gerührt. Die fertige Mischung liegt als Gel vor, wird abgekühlt und in ein Vorratsgefäß abgefüllt.

### Beispiel 3:

Man legt in einem Laborkneter 26,6g (w/w) Vistanex MM L-80 vor und granuliert das Material mit einer Vorlauftemperatur von 100°C. Nach 30 min gibt man 55,1g (w/w) Oppanol B 12 und 32,3g (w/w) Eastoflex PLS E 1003 zu. Die Masse wird 5 min homogenisiert. Dann gibt man 41,8g (w/w) pulvrige Cellulose (Elcema P050) zu und homogenisiert weitere 90 min bei einer Vorlauftemperatur von 70°C. Anschließend fügt man 9,5g (w/w) Oktyldodecanol, 15,2g (w/w) eines Gels aus PVP/Wasser 1:2 (w/w) und 9,5g (w/w) Harnstoff zu. Die Masse wird weitere 60min homogenisiert.
Die fertige Masse wird zwischen zwei silikonisierten Papieren in einer Hydraulikpresse bei 80°C ausgepresst. Dann kaschiert man eine PE-Folie als Trägermaterial zu und stanzt einzelne Pflaster aus.

### Beispiel 4:

Man legt in einem Laborkneter 26,6g (w/w) Vistanex MM L-80 vor und granuliert das Material mit einer Vorlauftemperatur von 100°C. Nach 30 min gibt man 55,1g (w/w) Oppanol B 12 und 32,3g (w/w) Eastoflex PLS E 1003 zu. Die Masse wird 5 min homogenisiert. Dann gibt man 41,8g (w/w) pulvrige Cellulose (Elcema P050) zu und homogenisiert weitere 90 min bei einer Vorlauftemperatur von 70°C. Anschließend fügt man 9,5g (w/w) Oktyldodecanol, 15,2g (w/w) eines Gels aus PVP/Wasser 1:2 (w/w) und 9,5g (w/w) Ascorbinsäure zu. Die Masse wird weitere 60min homogenisiert.
Die fertige Masse wird zwischen zwei silikonisierten Papieren in einer Hydraulikpresse bei 80°C ausgepresst. Dann kaschiert man eine PE-Folie als Trägermaterial zu und stanzt einzelne Pflaster aus.

### Beispiel 5:

Man legt in einem Laborkneter 38,0g (w/w) Oppanol B150 vor und granuliert das Material mit einer Vorlauftemperatur von 100°C. Nach 30 min gibt man 47,5g (w/w) Oppanol B 12 und 24,7g (w/w) Eastoflex PLS E 1003 zu. Die Masse wird 5 min homogenisiert. Dann gibt man 41,8g (w/w) pulvrige Cellulose (Elcema P050) zu und homogenisiert weitere 90 min bei einer Vorlauftemperatur von 70°C. Anschließend fügt man 9,5g (w/w) Oktyldodecanol, 19,0g (w/w) eines Gels aus PVP/Wasser 1:2 (w/w) und 9,5g (w/w) Dexpanthenol zu. Die Masse wird weitere 60min homogenisiert. Die fertige Masse wird zwischen zwei silikonisierten Papieren in einer Hydraulikpresse bei 80°C ausgepresst. Dann kaschiert man eine PE-Folie als Trägermaterial zu und stanzt einzelne Pflaster aus.

### Beispiel 6 (Extrusion)

Die Herstellung der Masse erfolgt in einem 50mm Leistritz Doppelschneckenextruder mit einem Masseausstoss von 40kg/h. Das Temperaturprofil für den Prozeß beträgt 130 - 70°C von der Aufgabezone bis zur Austragszone. Die Inhaltsstoffe der Klebmatrix werden nacheinander entlang der Gesamtlänge des Verfahrensteils in folgender Reihenfolge zudosiert:

| | | | |
|---|---|---|---|
| 1. | Oppanol B150: | 5,6kg/h | Feststoff granuliert |
| 2. | Oppanol B12: | 11,6kg/h | Schmelze |
| 3. | Eastoflex PLS E1003D: | 6,8kg/h | Schmelze |
| 4. | pulvrige Cellulose: | 8,8kg/h | Feststoff |
| 5. | Wasser/PVP Mischung | 3,2kg/h | flüssig |
| 6. | Oktyldodecanol | 2,0kg/h | flüssig |
| 7. | Dexpanthenol | 2,0kg/h | flüssig |

Die Masse wird kontinuierlich hergestellt und aus einer Breitschlitzdüse ausgetragen. Zwischen Austragszone und Breitschlitzdüse ist eine Zahnradpumpe installiert, die für einen gleichmäßigen Masseaustrag sorgt.
Die Klebmasse wird anschließend über einen Kalander zwischen Trägermaterial und einer Trennfolie beschichtet und zur weiteren Verarbeitung auf Rollen aufgewickelt.

### Beispiel 7

In Einem Getzmann-Mischer werden 450g Benzin (w/w) und 79,8g (w/w) Oppanol B150 vorgelegt und 30min bei 500U/min homogenisiert. Dann gibt man 165,3g (w/w) Oppanol B12 und 96,9g (w/w) Eastoflex PLS E 1003 zu und homogenisiert weitere 50min bei 1500U/min. Dabei wird der Vorlauf der Kühlung auf 25°C eingestellt. Anschließend gibt man 125,4g (w/w) pulvrige Cellulose zu und homogenisiert 70min bei 1500U/min. Dann gibt man 100g (w/w) Benzin, 28,5g (w/w) Oktyldodecanol, 45,6g (w/w) Wasser/PVP Gel und 28,5g (w/w) Dexpanthenol zu. Die Mischung wird 30min homogenisiert, dann gibt man erneut 50g (w/w) Benzin zu und homogenisiert 180min. Am Ende der Mischzeit wird das Material aus dem Mischgefäß entnommen und über einen Streichbalken auf Trennpapier mit einem Flächengewicht von z.B. 300g/m² (Trockengewicht) ausgestrichen. Der Film wird an der Luft getrocknet. Nach Abschluß des Trocknungsvorganges kaschiert man ein Viskosevlies zu und stanzt Pflaster aus.

## Patentansprüche

1. Polymermatrix, deren Polymere einen hydrophoben Charakter aufweisen, umfassend 0,1 bis 25 Gew.%, bezogen auf die Masse der Gesamtpolymermatrix, eines Mischsystems umfassend Pofy(1-vinyl-2-pyrrolidone) der allgemeinen Formel 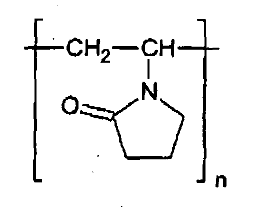 mit n = 350 bis 13500 und/oder deren Copolymere
und Wasser, wobei das Gewichtsverhältnis zwischen PVP und Wasser im Bereich von 1:5 bis 5:1, bevorzugt 1:3 bis 3:1, insbesondere 1:2 gewählt wird, **dadurch gekennzeichnet, dass** die Matrix als hydrophobe Basispolymere Styrol/Isopren/Styrol-Triblockcopolymere, Styrol/Butadien/Styrol-Triblockcopolymere, Copolymere aus Styrol und Butadien, Polyisobutylen, synthetische und/oder natürliche Polyisoprene, Polyester, Co-Polyester und/oder Mischungen daraus enthält oder daraus besteht.

2. Polymermatrix nach Anspruch 1, **dadurch gekennzeichnet, dass** die Matrix als hydrophobe Basispolymere Polyisobutylene enthält.

3. Polymermatrix nach einem der vorstehenden Ansprüche, **dadurch gekennzeichnet, dass** die Matrix lösemittelfrei ist.

4. Polymermatrix nach einem der vorstehenden Ansprüche enthaltend einen oder mehrere pharmazeutische Wirkstoffe und/oder Füllstoffe.

5. Polymermatrix nach Anspruch 4, **dadurch gekennzeichnet, dass** als Füllstoff Cellulose eingesetzt wird.

6. Polymermatrix nach Anspruch 4, **dadurch gekennzeichnet, dass** hydrophile Wirkstoffe enthalten sind.

7. Polymermatrix nach Anspruch 6, **dadurch gekennzeichnet, dass** als Wirkstoffe wasserlösliche Stoffe, Dexpanthenol, Ascorbinsäure und/oder Harnstoff eingebunden werden.

8. Polymermatrix nach Anspruch 4, **dadurch gekennzeichnet, dass** UV-Filter enthalten sind.

9. Polymermatrix nach einem der vorstehenden Ansprüche, **dadurch gekennzeichnet, dass** die Matrix transparent gestaltet ist.

10. Polymermatrix nach einem der vorstehenden Ansprüche, **dadurch gekennzeichnet, dass** die Matrix selbstklebend ausgerüstet ist.

11. Verwendung der Polymermatrix nach einem der Ansprüche 1 bis 10 als Pflaster, Pad oder Hautauflage.

12. Verwendung der Matrix nach einem der Ansprüche 1 bis 11 zur Herstellung eines Pflaster, Pad oder Hautauflage zur Hautpflege, zur Pflege sonnengeschädigter Haut oder zur Prophylaxe bei Sonnenexposition.

13. Verwendung der Matrix nach einem der Ansprüche 1 bis 11 zur Herstellung eines Pflaster, Pad oder Hautauflage zur Kühlung der Haut.

14. Verwendung der Matrix nach einem der Ansprüche 1 bis 11 zur Herstellung eines Pflaster, Pad oder Hautauflage zur Behandlung von trockener und/oder rissiger Haut.

15. Verwendung der Matrix nach einem der Ansprüche 1 bis 11 zur Herstellung eines Pflaster, Pad oder Hautauflage zur Beschleunigung der Wundheilung von verletzter Haut.

## Claims

1. Polymer matrix, the polymers of which have a hydrophobic character, comprising 0.1 to 25% by weight, based on the mass of the total polymer matrix, of a mixing system comprising poly(1-vinyl-2-pyrrolidones) of the general formula 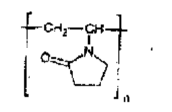 where n = 350 to 13 500 and/or copolymers thereof
and water, where the weight ratio between PVP and water is selected within the range from 1:5 to 5:1, preferably 1:3 to 3:1, in particular 1:2, **characterized in that** the matrix comprises, as hydrophobic base polymers, styrene/isoprene/styrene triblock copolymers, styrene/butadiene/styrene triblock copolymers, copolymers of styrene and butadiene, polyisobutylene, synthetic and/or natural polyisoprenes, polyesters, copolyester and/or mixtures thereof, or consists thereof.

2. Polymer matrix according to Claim 1, **characterized in that** the matrix comprises polyisobutylenes as hydrophobic base polymers.

3. Polymer matrix according to one of the preceding claims, **characterized in that** the matrix is solvent-free.

4. Polymer matrix according to one of the preceding claims, comprising one or more pharmaceutical active ingredients and/or fillers.

5. Polymer matrix according to Claim 4, **characterized in that** cellulose is used as filler.

6. Polymer matrix according to Claim 4, **characterized in that** hydrophilic active ingredients are present.

7. Polymer matrix according to Claim 6, **characterized in that** water-soluble substances, dexpanthenol, ascorbic acid and/or urea are incorporated as active ingredients.

8. Polymer matrix according to Claim 4, **characterized in that** UV filters are present.

9. Polymer matrix according to one of the preceding claims, **characterized in that** the matrix is transparent in design.

10. Polymer matrix according to one of the preceding claims, **characterized in that** the matrix has a self-adhesive finish.

11. Use of the polymer matrix according to one of Claims 1 to 10 as plaster, pad or skin covering.

12. Use of the matrix according to one of Claims 1 to 11 for producing a plaster, pad or skin covering for skin care, for the care of sun-damaged skin or for prophylaxis during exposure to the sun.

13. Use of the matrix according to one of Claims 1 to 11 for producing a plaster, pad or skin covering for cooling the skin.

14. Use of the matrix according to one of Claims 1 to 11 for producing a plaster, pad or skin covering for the treatment of dry and/or chapped skin.

15. Use of the matrix according to one of Claims 1 to 11 for producing a plaster, pad or skin covering for accelerating the wound healing of injured skin.

## Revendications

1. Matrice polymère, dont les polymères présentent un caractère hydrophobe, comprenant 0,1 à 25% en poids, par rapport à la masse de la matrice polymère totale, d'un système mixte comprenant de la poly(1-vinyl-2-pyrrolidone) de formule générale 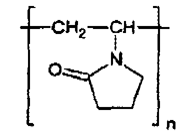 avec n = 350 à 13 500 et/ou ses copolymères
et de l'eau, le rapport pondéral entre la PVP et l'eau étant choisi dans la plage de 1:5 à 5:1, de préférence de 1:3 à 3:1, en particulier de 1:2, **caractérisée en ce que** la matrice contient ou est constituée par, comme polymères de base hydrophobes, des copolymères à trois blocs de styrène/isoprène/styrène, des copolymères à trois blocs de styrène/butadiène/styrène, des copolymères de styrène et de butadiène, du polyisobutylène, des polyisoprènes naturels et/ou synthétiques, des polyesters, des co-polyesters et/ou leurs mélanges.

2. Matrice polymère selon la revendication 1, **caractérisée en ce que** la matrice contient des polyisobutylènes comme polymères de base hydrophobes.

3. Matrice polymère selon l'une quelconque des revendications précédentes, **caractérisée en ce que** la matrice est exempte de solvants.

4. Matrice polymère selon l'une quelconque des revendications précédentes, contenant une ou plusieurs substances actives pharmaceutiques et/ou charges.

5. Matrice polymère selon la revendication 4, **caractérisée en ce que** de la cellulose est utilisée comme charge.

6. Matrice polymère selon la revendication 4, **caractérisée en ce que** des substances actives hydrophiles sont contenues.

7. Matrice polymère selon la revendication 6, **caractérisée en ce que** des substances solubles dans l'eau, le Dexpanthénol, l'acide ascorbique et/ou l'urée sont liées comme substances actives.

8. Matrice polymère selon la revendication 4, **caractérisée en ce que** des filtres UV sont contenus.

9. Matrice polymère selon l'une quelconque des revendications précédentes, **caractérisée en ce que** la matrice a un aspect transparent.

10. Matrice polymère selon l'une quelconque des revendications précédentes, **caractérisée en ce que** la matrice est apprêtée de manière autoadhésive.

11. Utilisation de la matrice polymère selon l'une quelconque des revendications 1 à 10 comme pansement, comme compresse ou comme patch transdermique.

12. Utilisation de la matrice selon l'une quelconque des revendications 1 à 11 pour la réalisation d'un pansement, d'une compresse ou d'un patch transdermique, pour le soin de la peau abîmée par le soleil ou pour la prophylaxie lors de l'exposition au soleil.

13. Utilisation de la matrice selon l'une quelconque des revendications 1 à 11 pour la préparation d'un pansement, d'une compresse ou d'un patch transdermique pour refroidir la peau.

14. Utilisation de la matrice selon l'une quelconque des revendications 1 à 11 pour la préparation d'un pansement, d'une compresse ou d'un patch transdermique pour le traitement de la peau sèche et/ou crevassée.

15. Utilisation de la matrice selon l'une quelconque des revendications 1 à 11 pour la préparation d'un pansement, d'une compresse ou d'un patch transdermique pour accélérer la guérison des plaies d'une peau blessée.
